# FASCICULE DE BREVET EUROPEEN

(11) **EP 1 044 677 B1**
(45) Date de publication et mention de la délivrance du brevet: **14.11.2001**
(21) Numéro de dépôt: 00400763.9
(22) Date de dépôt: 20.03.2000
(51) Int. Cl.: A61K 7/48

(54) **Composition cosmétique comprenant l'association d'un ester particulier et d'un composé silicone**
Kosmetische Zusammensetzung enthaltend die Kombination von einem besonderen Ester und einer Silikonverbindung
Cosmetic composition containing the combination of a particular ester and a silicone compound

(30) Priorité: 16.04.1999 FR 9904818
(43) Date de publication de la demande: 18.10.2000
(73) Titulaire: L'OREAL, 75008 Paris (FR)
(72) Inventeur: Robert, Valérie, 75012 Paris (FR); Arnaud, Pascal, 94240 L'Hay-les-Roses (FR)
(74) Mandataire: Brédeville, Odile Marie

(56) Documents cités:
- PATENT ABSTRACTS OF JAPAN vol. 0171, no. 80 & JP 04 334309 A (LION), 20 novembre 1992 (1992-11-20)
- PATENT ABSTRACTS OF JAPAN vol. 10, no. 318 (C-381) [2374], 29 octobre 1986 (1986-10-29) & JP 61 130233 A (SHISEIDO), 18 juin 1986 (1986-06-18)

## Description

La présente invention a pour objet une composition cosmétique comprenant un ester particulier et un composé siliconé. Ces compositions peuvent constituer des compositions de maquillage, comme des poudres, des fards à paupière, des fonds de teint, des anticernes, des rouges à lèvres, des produits de maquillage pour le corps, des mascara, des eye-liners, ou encore des compositions de soin de la peau.

L'utilisation de composés siliconés associés à des composés hydrocarbonés dans des compositions cosmétiques, notamment de maquillage, est connue (voir abrégé de JP 04-334 309).

En effet, les composés siliconés permettent, entre autre, d'obtenir sur la peau un film particulièrement homogène, possédant de bonnes propriétés cosmétiques. Ils permettent également d'améliorer la tenue des compositions de fonds de teint ou de rouges à lèvres par exemple, et ce grâce à leur caractère hydrophobe. Dans les poudres cosmétiques, qui comprennent généralement une phase particulaire et une phase grasse à titre de liant, ils sont incorporés dans le liant pour apporter de la douceur.

Les composés hydrocarbonés sont également utilisés dans les compositions cosmétiques : dans le cas des poudres cosmétiques par exemple, il est connu d'utiliser comme liant un mélange d'huiles minérales et végétales associées à des esters pour obtenir des compositions adhérant bien sur la peau et résistantes aux chocs.

Ainsi, il est intéressant de disposer de compositions cosmétiques comprenant à la fois des composés siliconés et des composés hydrocarbonés. Toutefois, on a constaté que certains composés siliconés étaient incompatibles avec certains composés hydrocarbonés généralement utilisés dans les compositions cosmétiques, ce qui en limitait l'utilisation. En particulier, on constate de nombreux phénomènes de déphasage, de relargage ou encore de synérèse lorsque ces deux types de composés sont mis en présence l'un de l'autre.

Diverses solutions ont été proposées afin de permettre la préparation d'une composition cosmétique comprenant à la fois des composés siliconés et des composés hydrocarbonés. On peut citer, par exemple, l'utilisation de solvants ou co-solvants hydrocarbonés tels que les isoparaffines; toutefois, leur odeur et leur volatilité ne sont pas toujours appréciées.

Par ailleurs, les compositions cosmétiques comprenant ces agents solubilisants apportent, lorsqu'on les applique sur la peau, une sensation de sécheresse particulièrement désagréable, en particulier dans le cas des rouges à lèvres.

De plus, dans le cas des poudres cosmétiques par exemple, la composition finale doit être suffisamment homogène et compacte pour présenter une bonne aptitude au prélèvement et pour éviter par ailleurs une fragmentation provoquée notamment par des chocs : or ceci est rarement le cas lorsque la composition comprend un composé siliconé.

Ainsi, s'il est déjà connu d'utiliser l'association d'un composé siliconé et d'un composé hydrocarboné, en particulier un ester, dans les compositions cosmétiques en général, il est toujours difficile d'obtenir une composition comprenant ces deux types de composés qui soit homogène et stable, résistante aux chutes dans le cas d'une composition compactée et qui présente également de bonnes propriétés cosmétiques.

Le but de la présente invention est de proposer une composition cosmétique homogène, résistante et présentant de bonnes propriétés cosmétiques, comprenant au moins un composé siliconé en association avec un ester particulier.

La Demanderesse a trouvé de manière inattendue qu'en associant à un composé siliconé un ester spécifique, à savoir un ester d'acide gras ou d'alcool gras, la chaîne carbonée de l'acide ou alcool gras étant saturée et ramifiée et contenant 24 à 28 atomes de carbone, il était possible d'obtenir une composition cosmétique, qui non seulement est très homogène durablement, mais présente également d'excellentes propriétés cosmétiques et une excellente cohésion, notamment lorsque la composition est sous forme compactée.

L'invention a donc pour objet une composition cosmétique, en particulier de maquillage, caractérisée par le fait qu'elle comprend i) au moins un ester d'acide gras ou d'alcool gras, la chaîne carbonée de l'acide ou alcool gras étant saturée et ramifiée et contenant 24 à 28 atomes de carbone et ii) au moins un composé siliconé.

Les compositions selon l'invention sont homogènes : ainsi, en général, on observe une distribution homogène des constituants ; dans le cas de mélanges solide/liquide, tels que cire-dans-huile, on observe la formation d'un mélange homogène et limpide à chaud et la formation d'une dispersion homogène de la cire dans l'huile lors du refroidissement; dans le cas de sticks ou de poudres cosmétiques, on observe une bonne dispersion des pigments et dans le cas de compositions compactées ou sous forme de sticks, on observe une bonne cohésion du produit.

Les compositions selon l'invention restent homogènes même après application sur la peau et/ou les muqueuses, et ce pendant plusieurs heures.

Les compositions selon l'invention présentent également d'excellentes propriétés cosmétiques : elles sont très douces, elles adhèrent suffisamment à la peau et/ou aux muqueuses mais pas trop, elles s'appliquent facilement et procurent une sensation très agréable à l'application.

En particulier, en ce qui concerne les poudres cosmétiques, elles sont faciles à compacter, elles se délitent bien, elles présentent une bonne dureté. Les poudres compactées présentent une remarquable résistance aux chutes et un pourcentage de perte de produit après chute très faible.

Les compositions ne procurent pas de sensation de sécheresse lorsqu'elle sont appliquées sur la peau et/ou les muqueuses. Elles présentent une excellente tenue. Elles présentent également une excellente dispersion des pigments.

La présente invention a encore pour objet un procédé de traitement non thérapeutique de la peau et/ou des muqueuses, notamment un procédé de maquillage, consistant à appliquer sur la peau et/ou les muqueuses une composition telle que définie ci-dessus.

La présente invention a encore pour objet l'utilisation de l'association d'au moins un composé siliconé et d'au moins un ester d'acide gras ou d'alcool gras, la chaîne carbonée de l'acide ou alcool gras étant saturée et ramifiée et contenant 24 à 28 atomes de carbone, dans une composition cosmétique compacte, dans le but d'améliorer la résistance aux chocs de ladite composition.

La présente invention a également pour objet l'utilisation de l'association d'au moins un composé siliconé et d'au moins un ester d'acide gras ou d'alcool gras, la chaîne carbonée de l'acide ou alcool gras étant saturée et ramifiée et contenant 24 à 28 atomes de carbone, pour la préparation d'une composition compacte, dans le but d'améliorer la résistance aux chocs de ladite composition.

Les compositions selon l'invention trouvent notamment une application particulièrement intéressante dans le domaine du maquillage de la peau y compris du cuir chevelu, des muqueuses, des semi-muqueuses, et des phanères. On entend notamment par muqueuse, la partie interne de la paupière inférieure; par semimuqueuses, on entend plus particulièrement les lèvres du visage; par phanères, on entend les cils, sourcils, cheveux et ongles. Ainsi, l'invention trouve une application toute particulière dans le domaine des produits de soin et/ou de maquillage du visage et de la peau, tels que les fonds de teint, les rouges à lèvres, les anticemes, les fards à paupières, les poudres du visage et du corps, les produits de maquillage du corps.

D'autres caractéristiques, aspects et avantages de la présente invention apparaîtront à la lecture de la description détaillée qui va suivre.

Les compositions selon l'invention comprennent au moins un ester d'acide gras ou d'alcool gras, la chaîne carbonée de l'acide ou alcool gras étant saturée et ramifiée et contenant 24 à 28 atomes de carbone.

Le mot ester, selon l'invention, signifie un monoester ou un polyester. Par polyester, on entend au sens de la présente invention, un composé comprenant plus d'une fonction ester comme par exemple les diesters, les triesters, les tétraesters, etc... De préférence, l'ester selon l'invention est choisi parmi les polyesters. De préférence, l'ester selon l'invention comporte au moins 2 chaînes ramifiées en C₂₄ à C₂₈. Le mot ramifié signifie au moins une chaîne pendante hydrocarbonée comportant notamment de 1 à 14 atomes de carbone.

L'ester de l'invention comporte donc un reste d'alcool gras ou acide gras saturé en C₂₄ à C₂₈ notamment du type respectivement alcool gras de Guerbet de formule (a) ou acide gras de Guerbet de formule (b) suivantes : 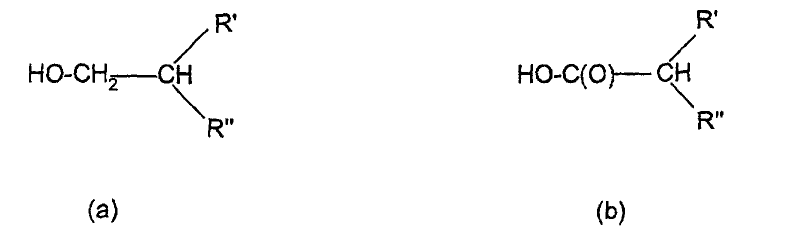 dans lesquelles R' et R" sont des radicaux alkyles saturés dont la somme des atomes de carbone va de 22 à 26. De préférence, le radical alkyle R" comprend deux atomes de carbone de moins que le radical alkyle R'.

De préférence, lorsque l'ester selon l'invention dérive d'un alcool gras de Guerbet, il ne dérive pas de l'acide benzoïque, et de préférence il dérive d'un acide gras aliphatique.

L'ester de la composition de l'invention est, de manière préférentielle, un ester huileux liquide à température ambiante (environ 25°C), présentant un poids moléculaire élevé, c'est-à-dire ayant un nombre de carbone supérieur ou égal à 50 et notamment supérieur ou égal à 70. L'intérêt d'un produit liquide par rapport à un produit pâteux ou solide à température ambiante, réside dans le nombre plus important de ses applications et sa facilité d'utilisation. De plus, le fait que cet ester présente un poids moléculaire élevé permet l'obtention de composition filmogène, rémanente à l'eau, ce qui est largement souhaité pour les produits de protection notamment solaire. Cet ester présente, entre autre, un indice de réfraction supérieur à 1,45 à 20°C et un indice d'iode ≤ 4.

Cet ester malgré son poids moléculaire élevé, n'est ni gras, ni lourd, ni collant et confère à la composition le contenant des propriétés de confort remarquable.

L'ester selon l'invention est avantageusement un ester d'acide gras ramifié en C₂₄-C₂₈ comme l'acide décyl 2-tétradécanoïque et plus spécialement un ester de polyol comme le glycérol, qui peut être un mono-, di- ou triglycéride. Préférentiellement, cet ester est un triglycéride d'acides gras ramifié en C₂₄-C₂₈ du type de Guerbet, et notamment un triglycéride d'acide gras en C₂₄ tel que l'acide décyl 2-tétradécanoïque.

De préférence, au moins une des chaînes ramifiées de l'ester contient 24 atomes de carbone.

De préférence encore, l'ester selon l'invention est choisi parmi les triglycérides d'acide gras ramifié en C24, les esters d'acide gras ramifié en C24 du pentaérythritol, les esters d'alcool gras ramifié en C24 et de diacides et leurs mélangés.

Un triglycéride préféré est par exemple le tri(décyl 2-tétradécanoate) de glycéryle vendu sous la référence DUB TGI 24 par la société Stearinerie Dubois. Cet ester présente un indice de saponification de 140 à 150, un indice de réfraction > à 1,45 et notamment allant de 1,454 à 1,459, à 20°C, un indice d'iode ≤ 4, un indice d'hydroxyle ≤ 30, un indice d'acide ≤ 10. Son nombre de carbone est de 75.

On peut aussi utiliser les esters d'acide gras en C₂₄ du pentaérythritol comme le tétra(décyl 2-tétradécanoate) de pentaérythrityle (à 101 atomes de carbone) vendu sous la référence DUB PTI 24 par la société Stéarinerie Dubois.

Lorsque l'alcool associé à un acide gras ramifié en C₂₄ à C₂₈ est un polyol, l'estérification peut être partielle (et concerner 1, 2, 3 ou plus de groupes OH selon l'alcool utilisé) ou être totale.

Comme ester de l'invention comportant un reste d'alcool gras à chaîne saturée ramifiée en C₂₄ à C₂₈, on peut citer les dimérates de di(décyltétradécyle) (à 84 atomes de carbone) comme celui vendu sous la référence DUB Dl 24D par la société Stéarinerie Dubois ou encore le néopentanoate de décyltétradécyle (à 29 atomes de carbone) ou encore l'isostéarate de décyl tétradécyle vendu par la société Condea sous la référence Isofol Ester 2482 (à 42 atomes de carbone). Les dimérates sont des esters issus de diacides, ces derniers étant obtenus généralement à partir d'acide insaturé en C₆ à C₂₄ comme l'acide oléïque, linoleïque, linolénique, etc...

De préférence, l'ester selon l'invention ne présente pas de propriétés tensioactives.

L'ester selon l'invention peut être présent à une teneur pouvant aller de 0,1 à 99,9% en poids, par rapport au poids total de la composition, et de préférence à une teneur allant de 1 à 99% en poids, par rapport au poids total de la composition.

Dans le cas d'une composition sous forme de produit pour le teint ou pour les lèvres fluide, l'ester est présent de préférence à une teneur allant de 1 à 99% en poids, par rapport au poids total de la composition. Dans le cas d'une composition sous forme de produit pour le teint ou pour les lèvres en stick, l'ester est présent de préférence à une teneur allant de 1 à 85% en poids, de préférence de 1 à 30%, en poids, par rapport au poids total de la composition.

Dans le cas d'une composition sous forme de poudre, l'ester selon l'invention représente de préférence de 0,1 à 23 % en poids, par rapport au poids total de la composition.

Les compositions selon l'invention comprennent en outre un composé siliconé.

Dans tout ce qui suit ou qui précède, on entend désigner par composé siliconé, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₃₀ et en particulier méthyle, les radicaux fluoroalkyles, les radicaux aryles et en particulier phényle.

Le composé siliconé peut être présent dans la composition à une teneur pouvant aller de 0,1 à 99,9%, en poids, de préférence de 1 à 99%, en poids, par rapport au poids total de la composition.

Le composé siliconé peut être choisi parmi les huiles de silicone, volatiles ou non, les cires de silicones, les gommes de silicone, les résines de silicone, les tensioactifs siliconés et/ou leurs mélanges.

Par huile de silicone, on entend, au sens de la présente invention, une silicone liquide à température ambiante (25°C), présentant de préférence une viscosité à 25°C allant de 0.005 - 5000 cm²/s (0,5 à 500 000 cst)(centistokes).

Comme huile de silicone convenant à la présente invention, on peut citer les polydiméthylsiloxanes (PDMS) linéaires répondant à la formule générale (I) suivante : 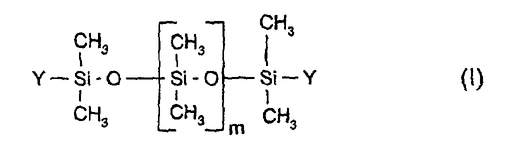 dans laquelle :
- Y est -CH₃ ou OH, et
- m est un entier allant de 0 à 2000.

Parmi ceux-ci, on citera notamment les produits vendus sous la dénomination de "AK" par la Société Wacker, "SF" par la Société Général Electric et "ABIL" par la Société Goldschmidt, tel que le produit "Abil 10", et les produits vendus sous la dénomination commerciale "DC 200" par Dow Corning.

On peut également citer :
- les polyalkyl(C₁-C₂₀) siloxanes et notamment ceux à groupements terminaux triméthylsilyle, de préférence ceux dont la viscosité est inférieure à 0,06 m²/s (60 000 cst) parmi lesquels on peut citer les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées.

On peut également citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la Silicone FZ 3109 vendue par la société Union Carbide , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (0.01 cm²/s (1 cst)à 25°C). On peut encore citer les alkyltrisiloxanes tels que l'hexylheptaméthyltrisiloxane ou l'octylheptaméthyltrisiloxane.

De préférence, on utilisera des huiles de silicone dont la viscosité à 25°C varie de 0.05 - 15cm²/s (5 cst à 1500 cst), et de préférence encore de 0.1 - 5 cm²/s (10 cst à 500 cst).

Ainsi, parmi les polyalkyl(C₁-C₂₀) siloxanes, on peut plus particulièrement citer la cétyl diméthicone vendue sous la dénomination commerciale "Abil wax 9801" par Goldschmidt et dont la viscosité à 25°C est de 0.15 - 0.25 cm²/s (15-25 cst).

Parmi les silicones phénylées, on peut plus particulièrement citer celles répondant à la formule (II) suivante : 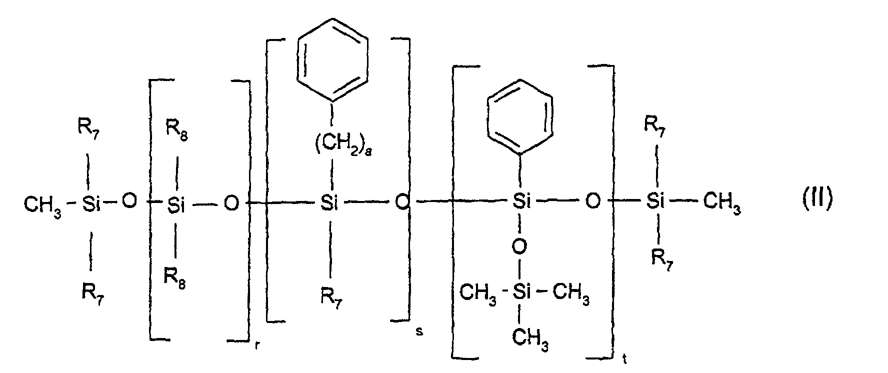 dans laquelle :
- R₇: représente un radical alkyl comportant de 1 à 30 atomes de carbone, ou un radical aryl, de préférence phényl, ou un radical aralkyl.
- R₈: représente un radical alkyl comportant de 1 à 30 atomes de carbone,
- r: est égal à 0 ou 1,
- s: représente un nombre entier compris entre 0 et 100,
- t: représente un nombre entier compris entre 0 et 100,
- a: représente un nombre entier compris entre 0 et 10,
sous réserve que la somme r + s + t soit comprise entre 1 et 100 et que si s=t=0, alors R₇ représente un radical aryle ou aralkyle.

Ainsi, parmi les silicones phénylées de formule (II), on peut citer le phényl triméthylsiloxytrisiloxane de viscosité 0.2 cm²/s (20 cst) à 25°C et vendu sous la dénomination commerciale "Dow Coming 556 Fluid" par la Société Dow Corning, le mélange de polydiphényl diméthylsiloxane et de phényltriméthicone de viscosité 0.14 cm²/s (14 cst) à 25°C vendu sous la dénomination commerciale "KF 56" par la Société Shin-Etsu, le diphénylméthyl diméthyl trisiloxane de viscosité 0.5 cm²/s (50 cst) à 25°C vendu sous la dénomination commerciale "DC-704" par la Société Dow Corning.

L'huile de silicone peut être présente dans les compositions de l'invention à une teneur pouvant aller de 1 à 99% en poids, par rapport au poids total de la composition.

Dans le cas d'une composition sous forme de produit pour le teint ou pour les lèvres fluide, l'huile de silicone est présente de préférence à une teneur allant de 1 à 99% en poids, par rapport au poids total de la composition. Dans le cas d'une composition sous forme de produit pour le teint ou pour les lèvres en stick, l'huile de silicone est présente de préférence à une teneur allant de 1 à 85% en poids, de préférence de 1 à 30% en poids, par rapport au poids total de la composition.

Dans le cas d'une composition sous forme de poudre, l'huile de silicone représente de préférence de 0,1 à 23 % en poids, par rapport au poids total de la composition.

Par cire de silicone, on entend, au sens de la présente invention, un composé siliconé solide ou pâteux à température ambiante (25°C), c'est-à-dire un composé présentant un point de fusion allant de 25 à 130 °C, de préférence de 30 à 105 °C.

De préférence, un composé pâteux aura un point de fusion allant de 25°C à 50°C et de préférence encore de 30°C à 50°C.

Un composé solide aura de préférence un point de fusion allant de 50°C à 130°C et de préférence encore de 50°C à 105°C.

Les cires de silicones utilisables selon la présente invention sont celles généralement utilisées en cosmétique. Parmi celles-ci, on peut plus particulièrement citer les organopolysiloxanes répondant à la formule (III) suivante : 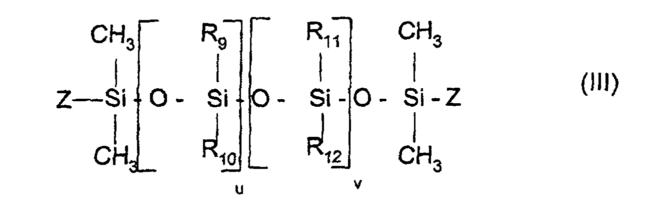 dans laquelle :
- R₉, R₁₁ et R₁₂ sont indépendamment les uns des autres, un radical alkyle ou alkoxy en C₁-C₃₀ ou un radical aryle en C₆-C₃₀,
- R₁₀ est un radical alkyle ou alkoxy en C₂-C₃₆ ou un radical alkyle ou alkoxy en C₂-C₃₆ substitué par un groupe ester, R₁₀ pouvant également être un radical méthyle lorsque Z est un radical alkyle ou alkoxy en C₂-C₃₀ ou un ester en C₂-C₃₀,
- Z est un radical alkyle ou alkoxy en C₁-C₃₀, un radical aryle en C₆-C₃₀ ou un ester en C₁-C₃₀;
- u est 0 ou un nombre entier entre 1 et 100,
- v est un nombre entier entre 1 et 100.

On peut citer par exemple la C₂₄-C₂₈ alkyl diméthicone vendue sous la dénomination commerciale "Abil Wax 9810" par la Société Goldschmidt, la stéaryldiméthicone vendue sous la dénomination commerciale "DC 2503" par la Société Dow Corning, la dibéhénoxydiméthicone vendue sous la dénomination commerciale "Abil Wax 2440" par la Société Goldschmidt ou encore la stéaroxydiméthicone vendue sous la dénomination commerciale "VP 1622" par la Société Wacker.

On peut également utiliser les organosiloxanes obtenus par action d'une cire naturelle, telle que la cire de Carnauba ou la cire d'abeilles, sur un squelette de silicone réactif.

La cire de silicone peut être présente à une teneur pouvant aller de 0,1 à 50% en poids, par rapport au poids total de la composition.

Dans le cas d'une composition sous forme de produit pour le teint ou pour les lèvres fluide, la cire de silicone est présente de préférence à une teneur allant de 0,1 à 5% en poids, par rapport au poids total de la composition. Dans le cas d'une composition sous forme de produit pour le teint ou pour les lèvres en stick, la cire de silicone est présente de préférence à une teneur allant de 0,1 à 50% en poids, par rapport au poids total de la composition.

Par gomme, on entend au sens de la présente invention un composé de viscosité à 25°C allant de 5000 - 5.10⁵ cm²/s (500 000 cst à 5 10⁷ cst) et qui, contrairement aux cires, ne présente pas de point de fusion.

On peut citer les gommes de silicone répondant à la formule (IV) suivante : 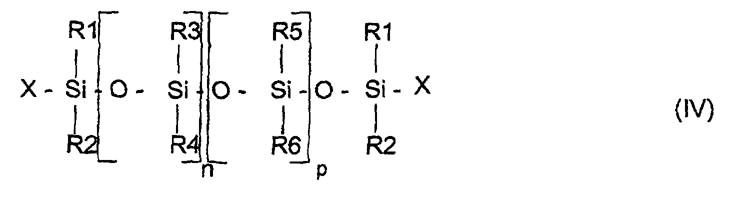 dans laquelle :
R₁, R₂, R₅ et R₆ sont, indépendamment les uns des autres, un radical alkyle ayant 1 à 6 atomes de carbone,
R₃ et R₄ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure a 5000 cm²/s (500 000 cst)

De manière générale, n et p peuvent prendre des valeurs de 0 à 5000, de préférence de 0 à 3000.

Comme gomme de silicone utilisable selon l'invention, on peut citer celles pour lesquelles :
- les substituants R1 à R6 et X représentent un groupement méthyle, p = 0 et n = 2700, comme celle vendue sous la dénomination SE30 par la société General Electric,
- les substituants R1 à R6 et X représentent un groupement méthyle, p = 0 et n = 2300, comme celle vendue sous la dénomination AK 500000 par la société Wacker,
- les substituants R1 à R6 représentent un groupement méthyle, le substituant X représente un groupement hydroxyle, p = 0 et n = 2700, en solution à 13 % dans du cyclopentasiloxane, comme celle vendue sous la dénomination Q2-1401 par la société Dow Corning,
- les substituants R1 à R6 représentent un groupement méthyle, le substituant X représente un groupement hydroxyle, p = 0 et n = 2700, en solution à 13% dans du polydiméthylsiloxane, comme celle vendue sous la dénomination Q2-1403 par la société Dow Corning,
- les substituants R1, R2, R5, R6 et X représentent un groupement méthyle, les substituants R3 et R4 représentent un groupement phényle tel que le poids moléculaire du composé soit de 600 000, comme celle vendue sous les dénominations "761" ou "Mirasil C-DPDM" par la société Rhône-Poulenc.

La gomme de silicone peut être présente à une teneur pouvant aller de 0,1 à 20% en poids, par rapport au poids total de la composition.

Dans le cas d'une composition sous forme de produit pour le teint ou pour les lèvres fluide, la gomme de silicone est présente de préférence à une teneur allant de 0,1 à 20% en poids, par rapport au poids total de la composition. Dans le cas d'une composition sous forme de produit pour le teint ou pour les lèvres en stick, la gomme de silicone est présente de préférence à une teneur allant de 0,1 à 10% en poids, par rapport au poids total de la composition.

Les résines de silicones sont des produits d'hydrolyse et de polycondensation de mélanges de siloxanes. Elles sont choisies parmi celles de formules R₃SiO_{1/2} , R₂SiO_{2/2} , RSiO_{3/2} et SiO_{4/2}, R représentant un groupement alkyle ayant de 1 à 6 atomes de carbone ou un groupe phényle. Ces résines peuvent être dissoutes dans un solvant tel qu'une huile polydiméthylsiloxane ou polyphénylsiloxane, ou une cyclométhicone. Ces résines peuvent être modifiées par un groupement ester comme dans le document EP-A-602 905 ou encore par un groupement alkyle.

Ces résines de silicone sont connues ou peuvent être préparées selon des méthodes connues.

Parmi les résines de silicone commerciales utilisables, on peut citer par exemple celles qui sont vendues sous les dénominations "DC 593" par Dow Coming ou "SS 4230" par General Electric.

La résine de silicone peut être présente à une teneur pouvant aller de 0,1 à 90% en poids, par rapport au poids total de la composition.

Dans le cas d'une composition sous forme de produit pour le teint ou pour les lèvres fluide, la résine de silicone est présente de préférence à une teneur allant de 0,1 à 70% en poids, par rapport au poids total de la composition. Dans le cas d'une composition sous forme de produit pour le teint ou pour les lèvres en stick, la résine de silicone est présente de préférence à une teneur allant de 0,1 à 50% en poids, par rapport au poids total de la composition.

Le composé siliconé de la présente invention peut également être un tensioactif siliconé, notamment une silicone oxyalkylénée.

Selon l'invention, on désigne par silicone oxyalkylénée toute silicone comportant au moins un groupement oxyalkyléné de type (-CₓH₂ₓO)ₐ dans lequel x peut varier de 2 à 6 et a est supérieur ou égal à 1.

On peut par exemple citer les produits vendus par la société Goldschmidt sous les dénominations commerciales ABIL WE 09, ABIL EM 90, ABIL B8852, par la société Dow Coming sous les dénominations DC 3225 C, Q2-5220, Q2-5200, par la société Amerchol sous la dénomination SILSOFT BEAUTY AID SL.

Le tensioactif siliconé peut être présent à une teneur pouvant aller de 0,1 à 40% en poids, par rapport au poids total de la composition.

Dans le cas d'une composition sous forme de produit pour le teint ou pour les lèvres fluide, le tensioactif siliconé est présent de préférence à une teneur allant de 0,1 à 40% en poids, par rapport au poids total de la composition. Dans le cas d'une composition sous forme de produit pour le teint ou pour les lèvres en stick, le tensioactif siliconé est présent de préférence à une teneur allant de 0,1 à 30% en poids, par rapport au poids total de la composition.

Outre l'ester particulier et le composé siliconé ci-dessus, la phase grasse de la composition selon l'invention peut comprendre tout autre corps gras comme des huiles et/ou des cires et/ou des corps gras pâteux non siliconés.

Ces corps gras peuvent être choisis parmi les huiles et/ou cires d'origine minérale, animale, végétale ou synthétique (dont les esters d'acides gras), les huiles fluorées.

Parmi les huiles utilisables, on peut citer l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline, les polyisobutènes hydrogénés et les polybutènes ; les esters gras, tels que le myristate d'isopropyle, le palmitate d'isopropyle, le stéarate de butyle, le laurate d'hexyle, l'isononanoate d'isononyle, le palmitate de 2-éthyl-hexyle, le laurate de 2-hexyl-décyle, le palmitate de 2-octyldécyle, le myristate ou le lactate de 2-octyl-dodécyle, le succinate de 2-diéthylhexyle, le malate de diisostéaryle, le triisostéarate de glycérine ou de diglycérine, les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'octyldodécanol.

Parmi les cires utilisables, on peut citer les cires d'abeille, les cires de lanoline et les cires d'insectes de Chine ; les cires de Camauba, de Candellila, d'Ouricury, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères et les esters cireux.

La composition de l'invention peut comprendre, en outre, tout additif usuellement utilisé dans le domaine concerné des antioxydants, des huiles essentielles, des conservateurs, des neutralisants, des tensio-actifs E/H ou H/E non siliconés, des vitamines, des actifs antirides.

La composition selon l'invention peut éventuellement comprendre des huiles volatiles autres que les huiles volatiles siliconées.

On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Ces composés volatils peuvent être choisis en particulier parmi les huiles hydrocarbonées telles que les isoparaffines et notamment l'isododécane.

La composition selon l'invention peut également comprendre une phase particulaire qui peut comprendre des composés pulvérulents choisis parmi les pigments et/ou les nacres et/ou les charges et/ou leurs mélanges habituellement utilisés dans les compositions cosmétiques.

Par pigments, il faut comprendre des particules blanches ou colorées, minérales ou organiques, insolubles dans le milieu, destinées à colorer et/ou opacifier la composition.

Les pigments peuvent être présents à raison de 0-40 % en poids, de préférence à raison de 0,1 à 30% en poids, par rapport au poids total de la composition et de préférence encore à raison de 1-20 %. Ils peuvent être blancs ou colorés, minéraux et/ou organiques, de taille usuelle ou nanométrique. On peut citer, parmi les pigments et les nanopigments minéraux, les dioxydes de titane de forme sphérique ou en paillettes, les dioxydes de zirconium ou de cérium, ainsi que les oxydes de zinc, de fer ou de chrome, les nanotitanes, le bleu ferrique. Parmi les pigments organiques, on peut citer le noir de carbone, et les laques couramment employées pour conférer aux lèvres et à la peau un effet de maquillage, qui sont des sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des PDMS et/ou par des polymères, notamment des polyéthylènes. On peut ainsi citer les pigments SA de Maprecos ou les pigments PI de Myoshi.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges peuvent être présentes dans la composition à raison de 0-99 % en poids, par rapport au poids total de la composition. Dans le cas d'une composition sous forme de poudre, les charges sont présentes de préférence à une teneur allant de 0,1 à 80 % en poids, par rapport au poids total de la composition. Dans le cas d'une composition sous forme de produit pour le teint ou pour les lèvres, les charges sont présentes de préférence à une teneur allant de 0,1 à 40% en poids, et de préférence encore à une teneur allant de 1 à 20% en poids, par rapport au poids total de la composition.

Les charges peuvent être minérales ou de synthèse, lamellaires ou non lamellaires. On peut citer le talc, le mica, la silice, le kaolin, les poudres de Nylon, de polyβ-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthane, les poudres de polystyrène, les poudres de polyester, les microsphères creuses synthétiques, les microéponges, les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, comme le stéarate de zinc, de magnésium ou de lithium, le laurate de zinc, le myristate de magnésium, les composés SiO₂/TiO₂/SiO₂, TiO₂/CeO₂/SiO₂, ou encore TiO₂/ZnO/Talc, les polymères de polyéthylène terephtalate/polyméthacrylate en forme de paillettes.

Par nacres, il faut comprendre des particules irisées qui réfléchissent la lumière.

Les nacres peuvent être présentes dans la composition à raison de 0-60% en poids, de préférence à raison de 0,1 à 40% en poids et de préférence encore à raison de 1-20% en poids, par rapport au poids total de la composition. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

En particulier, grâce à l'association selon l'invention, il est possible de réaliser des poudres compactes comprenant un taux élevé de nacre.

Les compositions de l'invention contiennent en outre un milieu cosmétiquement, physiologiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Lorsque les compositions de l'invention sont des poudres cosmétiques, elles comprennent de préférence de 77 à 99,9% en poids, par rapport au poids total de la composition, de phase particulaire et 0,1 à 23% de phase grasse. Elles peuvent se présenter sous la forme d'une poudre, par exemple compactée, libre, pressée ou encore coulée. Dans le cas d'une poudre libre par exemple, la phase grasse peut représenter de 1 à 5% en poids, par rapport au poids total de la composition. Pour une poudre compactée, la teneur en phase grasse peut représenter de 1 à 20% en poids, par rapport au poids total de la composition.

Lorsque la composition selon l'invention est sous forme de poudre, elle est de préférence sous forme de poudre compacte.

Lorsque les compositions de l'invention sont des produits pour le teint ou pour les lèvres, la phase grasse peut représenter de 5 à 100% en poids, de préférence de 20 à 95 % en poids, par rapport au poids total de la composition. Ces compositions peuvent se présenter sous la forme d'un gel anhydre.

Les compositions selon l'invention, en particulier lorsqu'elles se présentent sous la forme de produits pour le teint ou pour les lèvres, peuvent également comprendre une phase aqueuse.

Les compositions de l'invention peuvent ainsi se présenter sous la forme d'une émulsion huile-dans-eau (H/E), une émulsion eau-dans-huile (E/H), une émulsion multiple ou une solution multiphasée.

Les compositions de l'invention peuvent également se présenter sous la forme d'une dispersion vésiculaire, par exemple sous la forme d'une phase huileuse dispersée dans une phase aqueuse et stabilisée par des liposomes.

Lorsque la composition selon l'invention comprend une phase aqueuse, cette dernière peut être présente à une teneur allant de 0,1 à 95% en poids, par rapport au poids total de la composition. La phase aqueuse peut comprendre de l'eau, une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre en outre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition peut comprendre en outre tout composé complémentaire usuellement utilisé dans le domaine cosmétique, tel que des antioxydants, des parfums, des huiles essentielles, des conservateurs, des actifs cosmétiques ou pharmaceutiques lipophiles ou hydrophiles, des hydratants, des vitamines, des acides gras essentiels, des sphingolipides, des composés auto-bronzants tels que la DHA, des filtres solaires.

Ces composés complémentaires peuvent être présents dans la composition à raison de 0,1-20 % en poids, par rapport au poids total de la composition. Selon leur nature, ils sont présents dans la phase aqueuse ou dans la phase grasse de la composition.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les compositions selon l'invention peuvent être préparées selon les méthodes de préparation classiques des poudres, libres ou compactées, des émulsions E/H , H/E, des gels anhydres, sous forme de fluides, de crèmes, de compositions pâteuses ou de sticks, ces méthodes étant bien connues de l'homme du métier.

L'invention est illustrée plus en détails dans les exemples suivants.

Dans les exemples suivants, les quantités sont données en pourcentage en poids par rapport au poids total de la composition.

### EXEMPLE 1 : comparatif

La Demanderesse a réalisé les compositions A (selon l'invention) et B et C (comparatives) suivantes :
- Talc qsp 100%
- Mica 20 %
- BiOCl 10 %
- TᵢO₂ 2 %
- Sel métallique 3 %
- Pigments 15 %
- Nylon 20 %
- **Liant 5.5 %**
avec :

| **Composition** | **Composition du liant** (en % de poids, par rapport au poids total du liant) |
|---|---|
| Composition A (invention) | 50% tri(décyl 2-tetradécanoate) de glycéryle 50% polydiméthysiloxane 10 cst |
| Composition B (comparatif) | 50% triisostéarate de glycéryle 50% polydiméthysiloxane 10 cst |
| Composition C (comparatif) | 68% polydiméthylsiloxane 10 cst 13,2% polydiméthylsiloxane 100 cst 6,5% triméthylsiloxysilicate 12,3% polyméthylcétyl diméthylsiloxane 15-25 cst |
| (10 cst = 0.1 cm²/s ; 100 cst = 1 cm²/s ; 15 - 25 cst = 0.15 - 0.25 cm²/s) | |

Ces compositions ont été préparées de la manière suivante : les composés pulvérulents sont d'abord mélangés. Le liant est ajouté et on mélange à nouveau. Le tout est tamisé puis compacté dans une coupelle à une pression de compactage de 80 bars.

On obtient ainsi des poudres cosmétiques compactes qui peuvent par exemple être des fards à paupières.

Les compositions B et C comparatives comprennent chacune un liant selon l'art antérieur. Le liant présent dans B comprend un composé siliconé et du triisostéarate de glycéryle qui est un ester de structure chimique proche du tri(décyl 2-tétradécanoate) de glycéryle. Le liant présent dans C est un mélange de composés siliconés ne comprenant pas de composé hydrocarboné.

Les résistances aux chocs et les duretés respectives des compositions A, B et C ont été comparées.

La dureté de chaque composition a été mesurée à l'aide d'un duromètre Zwick gradué de 0 à 100 unités Shore A. On fait pénétrer l'aiguille du duromètre près du centre de la coupelle dans laquelle se trouve le produit à mesurer et on lit la dureté sur le cadran gradué.

Des tests de chute ont été réalisés sur chacune des trois compositions selon le protocole suivant : on pèse chaque échantillon préalablement compacté dans une coupelle. Puis on fait chuter 10 fois chaque échantillon d'une hauteur de 20 cm, verticalement, sur une dalle en grès. On pèse à nouveau chaque échantillon et on calcule le pourcentage de produit perdu par rapport au poids du produit initial.

Les résultats sont rassemblés dans le tableau ci-dessous :

| Composition | % de perte de produit après chute | Dureté (° Shore) |
|---|---|---|
| Composition A (invention) | 6% | 55 |
| Composition B (comparatif) | 14,3% | 58 |
| Composition C (comparatif) | 28% | 55 |

Ainsi, pour une dureté équivalente, la composition selon l'invention, qui comprend l'association "ester + polydiméthylsiloxane" conforme à la présente invention, présente de meilleurs résultats, en test de chute, qu'une composition comprenant une association "ester + polydiméthysiloxane" non conforme à la présente invention ou qu'une composition comprenant seulement un mélange de composés siliconés. La composition A selon l'invention présente donc une meilleure cohésion que les compositions de l'art antérieur.

### EXEMPLE 2 : comparatif

Des compositions **D** et **E** ont été réalisées correspondant aux compositions de l'exemple 1 mais pour lesquelles le liant est :
- composition **D** : tri(décyl 2-tetradécanoate) de glycéryle seul,
- composition **E** : polydiméthylsiloxane seul.

Les compositions A, B, C, D et E ont été respectivement appliquées sur les deux paupières de cinq personnes. Les critères suivants ont ensuite été évalués :
- l'adhérence
- la douceur
- la facilité d'application
- l'homogénéité
- l'appréciation globale

II en ressort que la composition A qui comprend l'association selon l'invention est supérieure globalement, en ce qui concerne les qualités cosmétiques citées ci-dessus, aux compositions B, C, D et E.

### EXEMPLE 3 :

La Demanderesse a réalisé les compositions F selon l'invention et G (comparative) suivantes :
- Talc qsp 100%
- Mica 10 %
- BiOCI 3 %
- TᵢO₂ 3 %
- Sel métallique 2 %
- Pigments 4 %
- Nylon 10 %
- Nacres 50 %
- **liant** 18 %
avec :

| **Composition** | **Composition du liant (en % de poids, par rapport au poids total du liant)** |
|---|---|
| Composition F (invention) | 50% tri(décyl 2-tetradécanoate) de glycéryle 50% cétyl diméthicone 0.15 - 0.25 cm²/s (15-25 cst) |
| Composition G (comparatif) | 100% tri(décyl 2-tetradécanoate) de glycéryle |

Ces compositions ont été préparées selon la méthode de préparation de l'exemple 1 mais ont été compactées à une pression de compactage de 130 bars. Malgré la présence de 50% de nacres, il est possible de les compacter.

La composition F présente une bonne cohésion.

Par ailleurs, les qualités cosmétiques de ces deux compositions ont été comparées comme dans l'exemple 2. Il en ressort que, pour 75% des personnes interrogées, la composition F selon l'invention est plus douce, plus facile à appliquer, plus homogène et plus agréable globalement que la composition G.

### EXEMPLE 4 :

La demanderesse a réalisé le rouge à lèvre en stick suivant :
- cire de polyéthylène vendue sous la dénomination commerciale "Performalene 500" par le société New Phase Technologies 11 %

### Phase A

- phényltriméthicone vendue sous la dénomination commerciale "DC 556" par Dow Coming 35,10 %
- tri(décyl 2-tétradécanoate) de glycéryle vendu sous la dénomination commerciale "DUB TGI 24" par la société Stéarinerie Dubois 35,11 %
- lanoline liquide 10,13 %

### Phase B

- FD et C Yellow n° 6 Al lake 3,30 %
- Oxyde de fer noir 0,06 %
- DC Red n° 21 Al. lake 0,60 %
- Dioxyde de titane 1,80 %
- DC Red n° 7 Ca. lake 2,90 %

### Mode opératoire :

Les constituants dé la phase A sont mélangés sous agitation magnétique à 60 °C. Après homogénéisation, on ajoute les pigments (phase B) et l'on broye l'ensemble à l'aide d'une broyeuse tricylindre.

On ajoute ensuite la cire et on chauffe le mélange à 98 °C.

Après homogénéisation, à l'aide d'une agitation magnétique, on coule la composition à 98 °C dans un moule adéquat afin d'obtenir un stick de rouge à lèvres.

Le stick de rouge à lèvres ainsi réalisé est particulièrement homogène.

Par ailleurs, cette composition se caractérise par une grande douceur et une bonne brillance à l'application.

### EXEMPLE 5 :

La Demanderesse a réalisé le stick de rouge à lèvres H suivant :

### Composition H :

- cire de polyéthylène vendue sous la dénomination commerciale "Performalene 500" par le société New Phase Technologies 12 %
- Poudre de Nylon 5,00 %
- Cyclopentadiméthylsiloxane 34,34 %

### Phase A

- cire de silicone vendue sous la dénomination "DC 2503" par la Société Dow Coming 5,00 %
- phényltriméthicone vendue sous la dénomination "DC 556" par la Société Dow Coming 17,50 %
- tri(décyl 2-tétradécanoate) de glycéryle vendu sous la dénomination commerciale "DUB TGI 24" par la société Stéarinerie Dubois 17,50 %

### Phase B

- FD et C Yellow n° 6 Al lake 3,30 %
- Oxyde de fer noir 0,06 %
- DC Red n° 21 Al. lake 0,60 %
- Dioxyde de titane 1,80 %
- DC Red n° 7 ca. lake 2,90 %

### Mode opératoire :

Les constituants de la phase A sont mélangés sous agitation magnétique à 60 °C. Après homogénéisation, on ajoute les pigments (phase B) et l'on broye l'ensemble à l'aide d'une broyeuse tricylindre.

On ajoute ensuite la cire de polyéthylène et on chauffe le mélange à 98 °C.

Après homogénéisation, à l'aide d'une agitation magnétique, on ajoute la poudre de Nylon et le cyclopentadiméthylsiloxane.

Après homogénéisation, on coule la composition à 98 °C dans un moule adéquat afin d'obtenir un stick de rouge à lèvres.

La Demanderesse a ensuite réalisé la composition J identique à la composition H à la différence près que les 17,5% de tri(décyl 2-tétradécanoate) de glycéryle ont été remplacés par 17,5% de phényl triméthicone.

Les compositions H et J ont ensuite été comparées en ce qui concerne leurs propriétés cosmétiques. Les critères suivants ont ensuite été évalués :
- onctuosité à l'application,
- brillance à l'application,
- glissant à l'application,
- couvrance.
et ont été jugés supérieurs pour le stick H selon l'invention.

En particulier, le stick H présente une excellente dispersion des pigments dans la composition et donc une bonne homogénéité.

## Revendications

1. Composition cosmétique en particulier de maquillage, **caractérisée par le fait qu'**elle comprend i) au moins un ester d'acide gras ou d'alcool gras, la chaîne carbonée de l'acide ou alcool gras étant saturée et ramifiée et contenant 24 à 28 atomes de carbone et ii) au moins un composé siliconé.

2. Composition selon la revendication 1, **caractérisée par le fait que** l'ester comporte au moins 2 chaînes ramifiées en C₂₄ à C₂₈.

3. Composition selon l'une quelconque des revendications précédentes, **caractérisée par le fait que** l'ester a un nombre de carbone supérieur ou égal à 50, de préférence supérieur ou égal à 70.

4. Composition selon l'une quelconque des revendications précédentes, **caractérisée par le fait qu'**au moins une des chaînes ramifiées de l'ester contient 24 atomes de carbone.

5. Composition selon l'une quelconque des revendications précédentes, **caractérisée par le fait que** l'ester est choisi parmi les triglycérides d'acide gras ramifié en C24, les esters d'acide gras ramifié en C24 du pentaérythritol, les esters d'alcool gras ramifié en C24 et de diacides et leurs mélanges.

6. Composition selon l'une quelconque des revendications précédentes, **caractérisée par le fait que** l'ester présente un indice de réfraction supérieur à 1,45 à 20°C.

7. Composition selon l'une quelconque des revendications précédentes, **caractérisée par le fait que** l'ester présente un indice d'iode inférieur ou égal à 4.

8. Composition selon l'une quelconque des revendications précédentes, **caractérisée par le fait que** l'ester est le tri(décyl 2-tétradécanoate) de glycéryle.

9. Composition selon l'une quelconque des revendications précédentes, **caractérisée par le fait que** l'ester est présent à une teneur allant de 0,1 à 99,9% en poids, par rapport au poids total de la composition, et de préférence à une teneur allant de 1 à 99% en poids, par rapport au poids total de la composition.

10. Composition selon l'une quelconque des revendications précédentes, **caractérisée par le fait que** le composé siliconé est choisi parmi les huiles de silicone, volatiles ou non, les cires de silicones, les gommes de silicone, les résines de silicone, les tensioactifs siliconés et/ou leurs mélanges.

11. Composition selon l'une quelconque des revendications précédentes, **caractérisée par le fait que** le composé siliconé est présent dans la composition à une teneur pouvant aller de 0,1 à 99,9%, de préférence de 1 à 99%, en poids, par rapport au poids total de la composition.

12. Composition selon l'une quelconque des revendications précédentes, **caractérisée par le fait que** le composé siliconé est choisi parmi les huiles de silicone dont la viscosité à 25°C varie de 0.05 - 15 cm²/s (5 cst à 1500 cst), et de préférence encore de 0.1 - 5 cm²/s (10 cst à 500 cst).

13. Composition selon l'une quelconque des revendications précédentes, **caractérisée par le fait que** le composé siliconé est choisi parmi les huiles de silicone phénylée répondant à la formule (II) suivante : 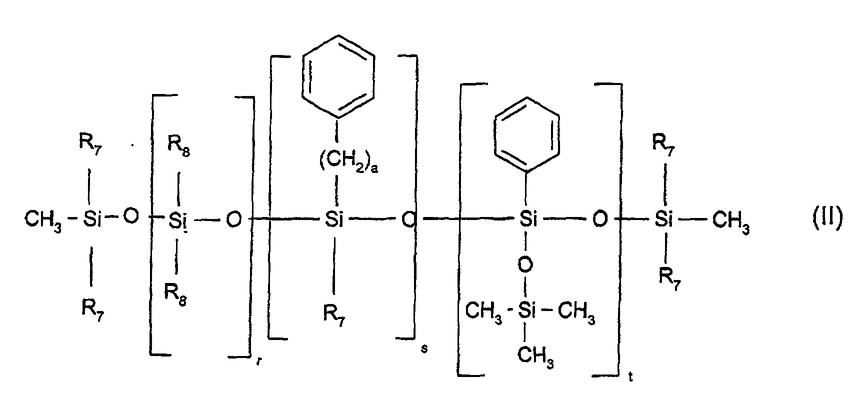 dans laquelle :
R₇ représente un radical alkyl comportant de 1 à 30 atomes de carbone, ou un radical aryl, de préférence phényl, ou un radical aralkyl.
R₈ représente un radical alkyl comportant de 1 à 30 atomes de carbone,
r est égal à 0 ou 1,
s représente un nombre entier compris entre 0 et 100,
t représente un nombre entier compris entre 0 et 100,
a représente un nombre entier compris entre 0 et 10,
sous réserve que la somme r + s + t soit comprise entre 1 et 100 et que si s=t=0, alors R₇ représente un radical aryle ou aralkyle.

14. Composition selon l'une quelconque des revendications 10 à 13, **caractérisée par le fait que** l'huile siliconée volatile est choisie parmi les silicones volatiles cycliques ayant de 3 à 8 atomes de silicium, les cyclocopolymères du type diméthylsiloxane/méthylalkylsiloxane, les silicones volatiles linéaires ayant de 2 à 9 atomes de silicium.

15. Composition selon l'une quelconque des revendications 10 à 14, **caractérisée par le fait que** l'huile de silicone est présente à une teneur allant de 1 à 99% en poids, par rapport au poids total de la composition.

16. Composition selon l'une quelconque des revendications 10 à 15, **caractérisée par le fait que** le composé siliconé est choisi parmi les cires de silicone répondant à la formule (III) suivante : 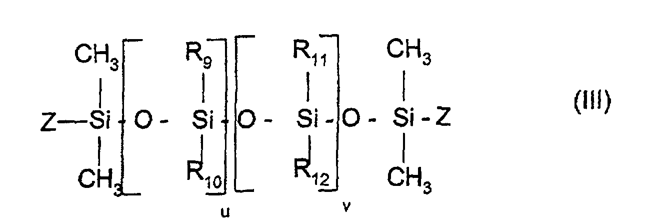 dans laquelle :
- R₉, R₁₁ et R₁₂ sont indépendamment les uns des autres, un radical alkyle ou alkoxy en C₁-C₃₀ ou un radical aryle en C₆-C₃₀,
- R₁₀ est un radical alkyle ou alkoxy en C₂-C₃₆ ou un radical alkyle ou alkoxy en C₂-C₃₆ substitué par un groupe ester, R₁₀ pouvant également être un radical méthyle lorsque Z est un radical alkyle ou alkoxy en C₂-C₃₀ ou un ester en C₂-C₃₀,
- Z est un radical alkyle ou alkoxy en C₁-C₃₀, un radical aryle en C₆-C₃₀ ou un ester en C₁-C₃₀;
- u est 0 ou un nombre entier entre 1 et 100,
- v est un nombre entier entre 1 et 100.

17. Composition selon la revendication précédente, **caractérisée par le fait que** la cire de silicone est choisie parmi la C₂₄-C₂₈ alkyl diméthicone, la stéaryldiméthicone, la dibéhénoxydiméthicone, la stéaroxydiméthicone.

18. Composition selon la revendication précédente, **caractérisée par le fait que** la cire de silicone est présente à une teneur pouvant aller de 0,1 à 50% en poids, par rapport au poids total de la composition.

19. Composition selon l'une quelconque des revendications 10 à 18, **caractérisée par le fait que** le composé siliconé est choisi parmi les gommes de silicone répondant à la formule (IV) suivante : 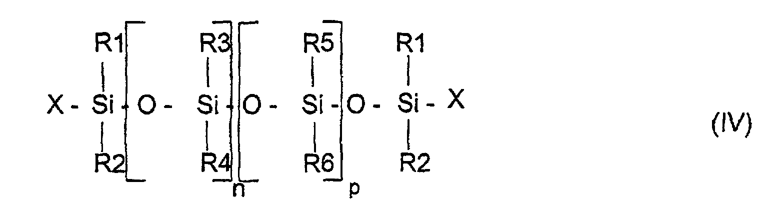 dans laquelle :
R₁, R₂, R₅ et R₆ sont, indépendamment les uns des autres, un radical alkyle ayant 1 à 6 atomes de carbone,
R₃ et R₄ sont, ensemble ou séparément, un radical alkyle ayant de 1 à 6 atomes de carbone, ou un radical aryle et notamment phényle,
X est un radical alkyle ayant de 1 à 6 atomes de carbone, un radical hydroxyle ou un radical vinyle,
n et p étant choisis de manière à conférer à la gomme de silicone une viscosité supérieure à 5000 cm²/s (500 000 cst).

20. Composition selon la revendication précédente, **caractérisée par le fait que** la gomme de silicone est présente à une teneur allant de 0,1 à 20% en poids, par rapport au poids total de la composition.

21. Composition selon l'une quelconque des revendications 10 à 20, **caractérisée par le fait que** la résine de silicone est choisie parmi celles de formules R₃SiO_{1/2} , R₂SiO_{2/2} , RSiO_{3/2} et SiO_{4/2}, R représentant un groupement alkyle ayant de 1 à 6 atomes de carbone ou un groupe phényle.

22. Composition selon la revendication précédente, **caractérisée par le fait que** la résine de silicone est présente à une teneur pouvant aller de 0,1 à 90% en poids, par rapport au poids total de la composition.

23. Composition selon l'une quelconque des revendications 10 à 22, **caractérisée par le fait que** le tensioactif siliconé est choisi parmi les silicones comportant au moins un groupement oxyalkyléné de type (-CₓH₂ₓO)ₐ dans lequel x peut varier de 2 à 6 et a est supérieur ou égal à 1.

24. Composition selon la revendication précédente, **caractérisée par le fait que** le tensioactif siliconé est présent à une teneur allant de 0,1 à 40% en poids, par rapport au poids total de la composition.

25. Composition selon l'une quelconque des revendications précédentes, **caractérisée par le fait qu'**elle comprend en outre une huile choisie parmi l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ; les huiles d'hydrocarbures, telles que les huiles de paraffine, le squalane, la vaseline, les polyisobutènes hydrogénés et les polybutènes ; les esters gras, tels que le myristate d'isopropyle, le palmitate d'isopropyle, le stéarate de butyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-diéthyl-hexyle, le malate de diisostéaryle, le triisostéarate de glycérine ou de diglycérine, les huiles perfluorées ; les acides gras supérieurs tels que l'acide myristique, l'acide palmitique, l'acide stéarique, l'acide béhénique, l'acide oléique, l'acide linoléique, l'acide linolénique ou l'acide isostéarique ; les alcools gras supérieurs tels que le cétanol, l'alcool stéarylique ou l'alcool oléique, l'octyldodécanol.

26. Composition selon l'une quelconque des revendications précédentes, **caractérisée par le fait qu'**elle comprend en outre une cire choisie parmi les cires d'abeille, les cires de lanoline et les cires d'insectes de Chine ; les cires de Camauba, de Candellila, d'Ouricury, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée ; les paraffines, les cires microcristallines, les cires de Montan et les ozokérites ; les cires de polyéthylène, les cires obtenues par la synthèse de Fisher-Tropsch, les copolymères et les esters cireux.

27. Composition selon l'une quelconque des revendications précédentes, **caractérisée par le fait qu'**elle comprend en outre une phase particulaire.

28. Composition selon la revendication précédente, **caractérisée par le fait que** la phase particulaire comprend des composés pulvérulents choisis parmi les pigments et/ou les nacres et/ou les charges et/ou leurs mélanges.

29. Composition selon la revendication précédente, **caractérisée par le fait que** les pigments sont choisis parmi les dioxydes de titane, de forme sphérique ou en paillettes, les dioxydes de zirconium ou de cérium, les oxydes de zinc, de fer ou de chrome, les nanotitanes, le bleu ferrique, le noir de carbone, les sels de calcium, de baryum, d'aluminium ou de zirconium, de colorants acides tels que les colorants halogéno-acides, azoïques ou anthraquinoniques, les pigments enrobés par des composés siliconés tels que des PDMS et/ou par des polymères.

30. Composition selon la revendication précédente, **caractérisée par le fait que** les pigments sont présents à raison de 0-40 % en poids, de préférence à raison de 0,1 à 30% en poids, par rapport au poids total de la composition.

31. Composition selon l'une quelconque des revendications 28 à 30, **caractérisée par le fait que** les charges sont choisies parmi le talc, le mica, la silice, le kaolin, les poudres de Nylon, de poly-β-alanine et de polyéthylène, le Téflon, la lauroyllysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges, les microbilles de résine de silicone, les oxydes de zinc et de titane, les oxydes de zirconium ou de cérium, le carbonate de calcium précipité, le carbonate et l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, comme le stéarate de zinc, de magnésium ou de lithium, le laurate de zinc, le myristate de magnésium, les composés SiO₂/TiO₂/ SiO₂, TiO₂/CeO₂/SiO₂, ou encore TiO₂/ZnO/Talc, les polymères de polyéthylène terephtalate/polyméthacrylate en forme de paillettes.

32. Composition selon la revendication précédente, **caractérisée par le fait que** les charges sont présentes à raison de 0-99 % en poids, par rapport au poids total de la composition.

33. Composition selon l'une quelconque des revendications 28 à 32, **caractérisée par le fait que** les nacres sont choisies parmi la nacre naturelle, le mica recouvert d'oxyde de titane, d'oxyde de fer, de pigment naturel ou d'oxychlorure de bismuth ainsi que le mica titane coloré.

34. Composition selon la revendication précédente, **caractérisée par le fait que** les nacres sont présentes dans la composition à raison de 0 à 60% en poids et de préférence encore à raison de 0,1 à 40% en poids, par rapport au poids total de la composition.

35. Composition selon l'une quelconque des revendications précédentes, **caractérisée par le fait qu'**elle comprend en outre une phase aqueuse à une teneur allant de 0,1 à 95% en poids, par rapport au poids total de la composition.

36. Produit pour le teint ou pour les lèvres comprenant une composition telle que définie à l'une quelconque des revendications 1 à 35.

37. Produit selon la revendication 36, **caractérisée par le fait que** la cire est présente à une teneur allant de 0,1 à 5% en poids, par rapport au poids total du produit.

38. Produit selon la revendication précédente, **caractérisé par le fait qu'**il est fluide.

39. Produit selon l'une quelconque des revendications 36 à 38, **caractérisé par le fait que** la résine de silicone est présente de préférence à une teneur allant de 0,1 à 70% en poids, par rapport au poids total du produit.

40. Produit selon l'une quelconque des revendications 36 à 39, **caractérisé par le fait que** les charges sont présentes de préférence à une teneur allant de 0,1 à 40% en poids, et de préférence encore à une teneur allant de 1 à 20% en poids, par rapport au poids total du produit.

41. Stick pour le teint ou pour les lèvres, **caractérisé par le fait qu'**il comprend un produit tel que défini à l'une quelconque des revendications 36 à 40.

42. Stick selon la revendication 41, **caractérisé par le fait que** l'ester est présent à une teneur allant de 1 à 85 % en poids, de préférence de 1 à 30 % en poids, par rapport au poids total du stick.

43. Stick selon la revendication 41 ou 42, **caractérisé par le fait que** l'huile de silicone est présente à une teneur allant de 1 à 85 % en poids, de préférence de 1 à 30 % en poids, par rapport au poids total du stick.

44. Stick selon l'une quelconque des revendications 41 à 43, **caractérisé par le fait que** la gomme de silicone est présente à une teneur allant de 0,1 à 10 % en poids, par rapport au poids total du stick.

45. Stick selon l'une quelconque des revendications 41 à 44, **caractérisé par le fait que** la résine de silicone est présente à une teneur allant de 0,1 à 50 % en poids, par rapport au poids total du stick.

46. Stick selon l'une quelconque des revendications 41 à 45, **caractérisé par le fait que** le tensioactif siliconé est présent à une teneur allant de 0,1 à 30 % en poids, par rapport au poids total du stick.

47. Poudre cosmétique comprenant une composition telle que définie à l'une quelconque des revendications 1 à 35.

48. Poudre selon la revendication précédente, **caractérisée par le fait que** l'ester est présent à une teneur allant de 0,1 à 23 % en poids, par rapport au poids total de la poudre.

49. Poudre selon la revendication 47 ou 48, **caractérisée par le fait que** l'huile de silicone est présente à une teneur allant de 0,1 à 23 % en poids, par rapport au poids total de la poudre.

50. Poudre selon l'une quelconque des revendications 47 à 49, **caractérisée par le fait que** les charges sont présentes à une teneur allant de 0,1 à 80 % en poids, par . rapport au poids total de la poudre.

51. Poudre selon l'une quelconque des revendications 47 à 50, **caractérisée par le fait que** la phase particulaire est présente à une teneur allant de 77 à 99,9 % en poids, par rapport au poids total de la poudre.

52. Poudre selon l'une quelconque des revendications 47 à 51, **caractérisée par le fait qu'**elle est sous forme de poudre compacte.

53. Procédé de traitement non thérapeutique de la peau et/ou des muqueuses, notamment un procédé de maquillage, consistant à appliquer sur la peau et/ou les muqueuses une composition telle que définie à l'une quelconque des revendications 1 à 35 et/ou un produit tel que défini à l'une quelconque des revendications 36 à 40 et/ou un stick tel que défini à l'une quelconque des revendications 41 à 46 et/ou une poudre telle que définie à l'une quelconque des revendications 47 à 52.

54. Utilisation de l'association d'au moins un composé siliconé et d'au moins un ester d'acide gras ou d'alcool gras, la chaîne carbonée de l'acide ou alcool gras étant saturée et ramifiée et contenant 24 à 28 atomes de carbone, dans une composition cosmétique compacte, dans le but d'améliorer la résistance aux chocs de ladite composition.

55. Utilisation de l'association d'au moins un composé siliconé et d'au moins un ester d'acide gras ou d'alcool gras, la chaîne carbonée de l'acide ou alcool gras étant saturée et ramifiée et contenant 24 à 28 atomes de carbone, pour la préparation d'une composition compacte, dans le but d'améliorer la résistance aux chocs de ladite composition.

## Patentansprüche

1. Kosmetische Zusammensetzung insbesondere zum Schminken, **dadurch gekennzeichnet, daß** sie i) mindestens einen Ester einer Fettsäure oder eines Fettalkohols, wobei die Kohlenstoffkette der Fettsäure oder des Fettalkohols gesättigt und verzweigt vorliegt und 24 bis 28 Kohlenstoffatome aufweist, und ii) mindestens eine Siliconverbindung enthält.

2. Zusammensetzung nach Anspruch 1, **dadurch gekennzeichnet, daß** der Ester mindestens 2 verzweigte C₂₄₋₂₈-Ketten aufweist.

3. Zusammensetzung nach einem der vorhergehenden Ansprüche, **dadurch gekennzeichnet, daß** die Anzahl der Kohlenstoffatome in dem Ester mindestens 50 und vorzugsweise mindestens 70 beträgt.

4. Zusammensetzung nach einem der vorhergehenden Ansprüche, **dadurch gekennzeichnet, daß** mindestens eine Kette der verzweigten Ketten des Esters 24 Kohlenstoffatome aufweist.

5. Zusammensetzung nach einem der vorhergehenden Ansprüche, **dadurch gekennzeichnet, daß** der Ester unter den Triglyceriden einer verzweigten C₂₄-Fettsäure, den Estern einer verzweigten C₂₄-Fettsäure und Pentaerythrit, den Estern eines verzweigten C₂₄-Fettalkohols und Disäuren und deren Gemischen ausgewählt ist.

6. Zusammensetzung nach einem der vorhergehenden Ansprüche, **dadurch gekennzeichnet, daß** der Ester bei 20 °C einen Brechungsindex über 1,45 aufweist.

7. Zusammensetzung nach einem der vorhergehenden Ansprüche, **dadurch gekennzeichnet, daß** der Ester eine Iodzahl von höchstens 4 hat.

8. Zusammensetzung nach einem der vorhergehenden Ansprüche, **dadurch gekennzeichnet, daß** der Ester das Glyceryl-tri(2-decyltetradecanoat) ist.

9. Zusammensetzung nach einem der vorhergehenden Ansprüche, **dadurch gekennzeichnet, daß** der Ester in einer Menge von 0,1 bis 99,9 Gew.-%, bezogen auf das Gesamtgewicht der Zusammensetzung, und vorzugsweise in einer Menge von 1 bis 99 Gew.-%, bezogen auf das Gesamtgewicht der Zusammensetzung, vorliegt.

10. Zusammensetzung nach einem der vorhergehenden Ansprüche, **dadurch gekennzeichnet, daß** die Siliconverbindung unter den flüchtigen oder nicht flüchtigen Siliconölen, Siliconwachsen, Silicongummis, Siliconharzen, siliconhaltigen grenzflächenaktiven Stoffen und/oder deren Gemischen ausgewählt ist.

11. Zusammensetzung nach einem der vorhergehenden Ansprüche, **dadurch gekennzeichnet, daß** die Siliconverbindung in der Zusammensetzung in einer Menge von 0,1 bis 99,9 % und vorzugsweise 1 bis 99 Gew.-%, bezogen auf das Gesamtgewicht der Zusammensetzung, vorliegt.

12. Zusammensetzung nach einem der vorhergehenden Ansprüche, **dadurch gekennzeichnet, daß** die Siliconverbindung unter den Siliconölen ausgewählt ist, die bei 25 °C eine Viskosität von 0,05 bis 15 cm²/s (5 bis 1500 cSt) und vorzugsweise 0,1 bis 5 cm²/s (10 bis 500 cSt) aufweisen.

13. Zusammensetzung nach einem der vorhergehenden Ansprüche, **dadurch gekennzeichnet, daß** die Siliconverbindung unter den phenylgruppenhaltigen Siliconölen der folgenden Formel (II) ausgewählt ist: 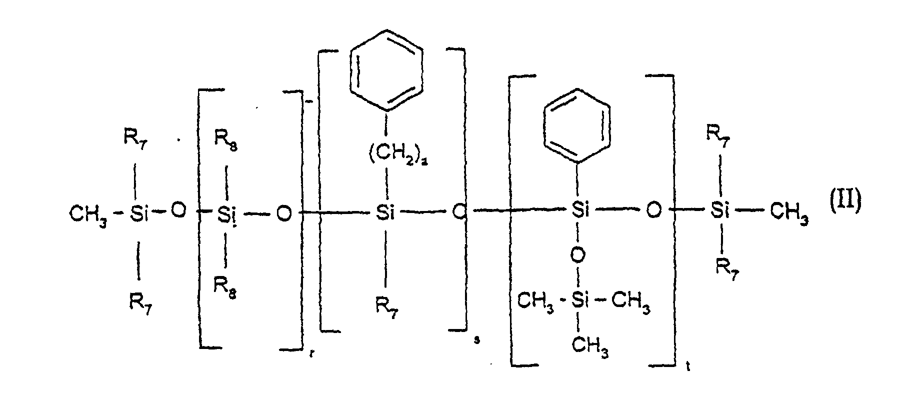 worin bedeuten:
R₇ eine Alkylgruppe mit 1 bis 30 Kohlenstoffatomen, eine Arylgruppe und vorzugsweise Phenyl oder eine Aralkylgruppe,
R₈ eine Alkylgruppe mit 1 bis 30 Kohlenstoffatomen,
r 0 oder 1,
s 0 oder eine ganze Zahl von 1 bis 100,
t 0 oder eine ganze Zahl von 1 bis 100,
a 0 oder eine ganze Zahl von 1 bis 10,
mit der Maßgabe, daß die Summe r + s + t im Bereich von 1 bis 100 liegt und daß R₇ eine Arylgruppe oder Aralkylgruppe bedeutet, wenn s = t = 0.

14. Zusammensetzung nach einem der Ansprüche 10 bis 13, **dadurch gekennzeichnet, daß** das flüchtige Siliconöl unter den cyclischen flüchtigen Siliconen mit 3 bis 8 Siliciumatomen, Cyclocopolymeren vom Typ Dimethylsiloxan/Methylalkylsiloxan und den geradkettigen flüchtigen Siliconen mit 2 bis 9 Siliciumatomen ausgewählt ist.

15. Zusammensetzung nach einem der Ansprüche 10 bis 14, **dadurch gekennzeichnet, daß** das Siliconöl in einer Menge von 1 bis 99 Gew.-%, bezogen auf das Gesamtgewicht der Zusammensetzung, vorliegt.

16. Zusammensetzung nach einem der Ansprüche 10 bis 15, **dadurch gekennzeichnet, daß** die Siliconverbindung unter den Siliconwachsen der folgenden Formel (III) ausgewählt ist: 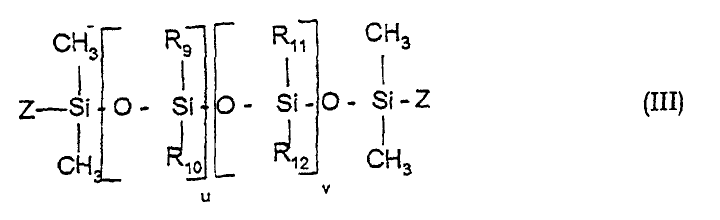 worin bedeuten:
- R₉, R₁₁ und R₁₂ unabhängig voneinander eine Alkyl- oder Alkoxygruppe mit 1 bis 30 Kohlenstoffatomen oder eine C₆₋₃₀-Arylgruppe,
- R₁₀ eine Alkyl- oder Alkoxygruppe mit 2 bis 36 Kohlenstoffatomen oder eine Alkyl- oder Alkoxygruppe mit 2 bis 36 Kohlenstoffatomen, die mit einer Estergruppe substituiert ist, wobei R₁₀ auch eine Methylgruppe bedeuten kann, wenn Z eine Alkyl- oder Alkoxygruppe mit 2 bis 30 Kohlenstoffatomen oder einen C₂₋₃₀-Ester bedeutet,
- Z eine Alkyl- oder Alkoxygruppe mit 1 bis 30 Kohlenstoffatomen, eine C₆₋₃₀-Arylgruppe oder eine C₁₋₃₀-Estergruppe,
- u 0 oder eine ganze Zahl von 1 bis 100,
- v eine ganze Zahl von 1 bis 100.

17. Zusammensetzung nach dem vorhergehenden Anspruch, **dadurch gekennzeichnet, daß** das Siliconwachs unter den C₂₄₋₂₈-Alkyldimethiconen, Stearyldimethicon, Dibehenoxydimethicon und Stearoxydimethicon ausgewählt ist.

18. Zusammensetzung nach dem vorhergehenden Anspruch, **dadurch gekennzeichnet, daß** das Siliconwachs in einer Menge von 0,1 bis 50 Gew.-%, bezogen auf das Gesamtgewicht der Zusammensetzung, vorliegt.

19. Zusammensetzung nach einem der Ansprüche 10 bis 18, **dadurch gekennzeichnet, daß** die Siliconverbindung unter den Silicongummis der folgenden Formel (IV) ausgewählt ist: 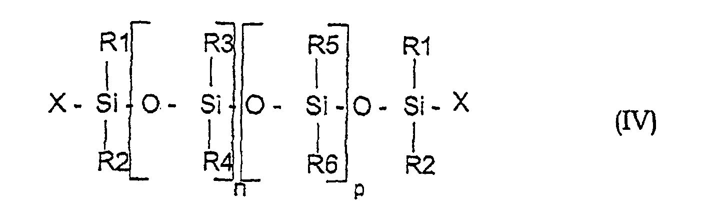 worin bedeuten:
R₁, R₂, R₅ und R₆ unabhängig voneinander eine Alkylgruppe mit 1 bis 6 Kohlenstoffatomen,
R₃ und R₄ gleichzeitig oder unabhängig voneinander eine Alkylgruppe mit 1 bis 6 Kohlenstoffatomen oder eine Arylgruppe, insbesondere Phenyl,
X eine Alkylgruppe mit 1 bis 6 Kohlenstoffatomen, eine Hydroxygruppe oder eine Vinylgruppe,
wobei n und p so ausgewählt sind, daß der Silicongummi eine Viskosität über 5000 cm²/s (500 000 cSt) aufweist.

20. Zusammensetzung nach dem vorhergehenden Anspruch, **dadurch gekennzeichnet, daß** der Silicongummi in einer Menge von 0,1 bis 20 Gew.-%, bezogen auf das Gesamtgewicht der Zusammensetzung, vorliegt.

21. Zusammensetzung nach einem der Ansprüche 10 bis 20, **dadurch gekennzeichnet, daß** das Siliconharz unter den Verbindungen der Formeln R₃SiO_{1/2}, R₂SiO_{2/2}, RSiO_{3/2} und SiO_{4/2} ausgewählt ist, wobei R eine Alkylgruppe mit 1 bis 6 Kohlenstoffatomen oder eine Phenylgruppe bedeutet.

22. Zusammensetzung nach dem vorhergehenden Anspruch, **dadurch gekennzeichnet, daß** das Siliconharz in einer Menge von 0,1 bis 90 Gew.-%, bezogen auf das Gesamtgewicht der Zusammensetzung, vorliegt.

23. Zusammensetzung nach einem der Ansprüche 10 bis 22, **dadurch gekennzeichnet, daß** der siliconhaltige grenzflächenaktive Stoff unter den Siliconen ausgewählt ist, die mindestens eine Alkylenoxidgruppe vom Typ (-CₓH₂ₓO)ₐ aufweisen, wobei x im Bereich von 2 bis 6 liegen kann und a mindestens 1 bedeutet.

24. Zusammensetzung nach dem vorhergehenden Anspruch, **dadurch gekennzeichnet, daß** der siliconhaltige grenzflächenaktive Stoff in einer Menge von 0,1 bis 40 Gew.-%, bezogen auf das Gesamtgewicht der Zusammensetzung, vorliegt.

25. Zusammensetzung nach einem der vorhergehenden Ansprüche, **dadurch gekennzeichnet, daß** sie außerdem ein Öl enthält, das ausgewählt ist unter: Nerzöl, Schildkrötenöl, Sojaöl, Traubenkernöl, Sesamöl, Maisöl, Rapsöl, Sonnenblumenöl, Baumwollöl, Avocadoöl, Olivenöl, Ricinusöl, Jojobaöl, Erdnußöl; Kohlenwasserstoffölen, beispielsweise Paraffinöl, Squalan, Vaseline, hydrierten Polyisobutenen und Polybutenen; Fettsäureestern, wie Isopropylmyristat, Isopropylpalmitat, Butylstearat, Hexyllaurat, Isononylisononanoat, 2-Ethylhexylpalmitat, 2-Hexyldecyllaurat, 2-Octyldecylpalmitat, 2-Octyldodecylmyristat, 2-Octyldodecyllactat, 2-Diethylhexylsuccinat, Diisostearylmalat, Glycerintriisostearat, Diglycerintriisostearat, perfluorierten Ölen; höheren Fettsäuren, wie Myristinsäure, Palmitinsäure, Stearinsäure, Behensäure, Ölsäure, Linolsäure, Linolensäure oder Isostearinsäure; höheren Fettalkoholen, wie Cetanol, Stearylalkohol, Oleylalkohol, Octyldodecanol.

26. Zusammensetzung nach einem der vorhergehenden Ansprüche, **dadurch gekennzeichnet, daß** sie außerdem ein Wachs enthält, das ausgewählt ist unter: Bienenwachs, Lanolinwachs, Chinawachs; Carnaubawachs, Candelillawachs, Ouricurywachs, Korkfaserwachsen, Zuckerrohrwachsen, Japanwachsen, hydrierten Jojobawachsen und hydrierten Ölen, wie hydriertem Sonnenblumenöl, hydriertem Ricinusöl, hydriertem Kopraöl und hydriertem Lanolin; Paraffinen, mikrokristallinen Wachsen, Montanwachsen und Ozokeriten; Polyethylenwachsen, durch Fischer-Tropsch-Synthese hergestellten Wachsen und wachsigen Copolymeren und Estern.

27. Zusammensetzung nach einem der vorhergehenden Ansprüche, **dadurch gekennzeichnet, daß** sie außerdem eine Partikelphase aufweist.

28. Zusammensetzung nach dem vorhergehenden Anspruch, **dadurch gekennzeichnet, daß** die Partikelphase Verbindungen in Pulverform enthält, die unter den Pigmenten und/oder Perlglanzpigmenten und/oder Füllstoffen und/oder deren Gemischen ausgewählt sind.

29. Zusammensetzung nach dem vorhergehenden Anspruch, **dadurch gekennzeichnet, daß** die Pigmente unter kugelförmig oder in Form von Plättchen vorliegendem Titandioxid, Zirconiumdioxid, Cerdioxid, den Oxiden von Zink, Eisen oder Chrom, Nanotitanen, Eisenblau, Ruß, Salzen von Calcium, Barium, Aluminium oder Zirconium, sauren Farbstoffen wie Halogensäure-Farbstoffen, Azofarbstoffen oder Anthrachinon-Farbstoffen und Pigmenten, die mit Siliconverbindungen wie PDMS und/oder Polymeren umhüllt sind, ausgewählt sind.

30. Zusammensetzung nach dem vorhergehenden Anspruch, **dadurch gekennzeichnet, daß** die Pigmente in einer Menge von 0 bis 40 Gew.-% und vorzugsweise 0,1 bis 30 Gew.-%, bezogen auf das Gesamtgewicht der Zusammensetzung, vorliegen.

31. Zusammensetzung nach einem der Ansprüche 28 bis 30, **dadurch gekennzeichnet, daß** die Füllstoffe ausgewählt sind unter: Talk, Glimmer, Kieselsäure, Kaolin, Nylonpulvern, Poly-βalaninpulvern und Polyethylenpulvern, Teflon, Lauroyllysin, Stärke, Bornitrid, Bismutoxidchlorid, Pulvern von Tetrafluorethylenpolymeren, Polymethylmethacrylatpulvern, Polyurethanpulvern, Polystyrolpulvern, Polyesterpulvern, synthetischen Mikrohohlkugeln, Mikroschwämmen, Siliconharzmikrokugeln, Oxiden von Zink und Titan, Oxiden von Zirconium oder Cer, gefälltem Calciumcarbonat, Magnesiumcarbonat, Magnesiumhydrogencarbonat, Hydroxyapatit, Siliciumoxidmikrohohlkugeln, Glasmikrokapseln, Keramikmikrokapseln, Metallseifen, die von organischen Carbonsäuren mit 8 bis 22 und vorzugsweise 12 bis 18 Kohlenstoffatomen, wie Zinkstearat, Magnesiumstearat, Lithiumstearat, Zinklaurat, Magnesiummyristat, den Verbindungen SiO₂/TiO₂/SiO₂, TiO₂/CeO₂/SiO₂ oder TiO₂/ZnO/Talk und den Polyethylenterephthalat/Polymethacrylat-Polymeren in Plättchenform.

32. Zusammensetzung nach dem vorhergehenden Anspruch, **dadurch gekennzeichnet, daß** die Füllstoffe in Mengen von 0 bis 99 Gew.-%, bezogen auf das Gesamtgewicht der Zusammensetzung, vorliegen.

33. Zusammensetzung nach einem der Ansprüche 28 bis 32, **dadurch gekennzeichnet, daß** die Perlglanzpigmente unter natürlichem Perlglanzpigment, mit Titanoxid, Eisenoxid, natürlichem Pigment oder Bismutoxidchlorid überzogenem Glimmer oder farbigem Titanglimmer ausgewählt sind.

34. Zusammensetzung nach dem vorhergehenden Anspruch, **dadurch gekennzeichnet, daß** die Perlglanzpigmente in der Zusammensetzung in einer Menge von 0 bis 60 Gew.-% und vorzugsweise 0,1 bis 40 Gew.-%, bezogen auf das Gesamtgewicht der Zusammensetzung, vorliegen.

35. Zusammensetzung nach einem der vorhergehenden Ansprüche, **dadurch gekennzeichnet, daß** sie außerdem eine wäßrige Phase in einer Menge von 0,1 bis 95 Gew.-%, bezogen auf das Gesamtgewicht der Zusammensetzung, enthält.

36. Produkt für den Teint oder für die Lippen, das eine Zusammensetzung nach einem der Ansprüche 1 bis 35 enthält.

37. Produkt nach Anspruch 36, **dadurch gekennzeichnet, daß** das Wachs in einer Menge von 0,1 bis 5 Gew.-%, bezogen auf das Gesamtgewicht des Produktes, vorliegt.

38. Produkt nach dem vorhergehenden Ansprüche, **dadurch gekennzeichnet, daß** es fluide ist.

39. Produkt nach einem der Ansprüche 36 bis 38, **dadurch gekennzeichnet, daß** das Siliconharz vorzugsweise in einer Menge von 0,1 bis 70 Gew.-%, bezogen auf das Gesamtgewicht des Produktes, vorliegt.

40. Produkt nach einem der Ansprüche 36 bis 39, **dadurch gekennzeichnet, daß** die Füllstoffe vorzugsweise in einer Menge von 0,1 bis 40 Gew.-% und noch bevorzugter in einer Menge von 1 bis 20 Gew.-%, bezogen auf das Gesamtgewicht des Produktes, vorliegen.

41. Stift für den Teint oder für die Lippen, **dadurch gekennzeichnet, daß** er ein Produkt nach einem der Ansprüche 36 bis 40 enthält.

42. Stift nach Anspruch 41, **dadurch gekennzeichnet, daß** der Ester in einer Menge von 1 bis 85 Gew.-% und vorzugsweise 1 bis 30 Gew.-%, bezogen auf das Gesamtgewicht des Stiftes, vorliegt.

43. Stift nach Anspruch 41 oder 42, **dadurch gekennzeichnet, daß** das Siliconöl in einer Menge von 1 bis 85 Gew.-% und vorzugsweise 1 bis 30 Gew.-%, bezogen auf das Gesamtgewicht des Stiftes, vorliegt.

44. Stift nach einem der Ansprüche 41 bis 43, **dadurch gekennzeichnet, daß** der Silicongummi in einer Menge von 0,1 bis 10 Gew.-%, bezogen auf das Gesamtgewicht des Stiftes, vorliegt.

45. Stift nach einem der Ansprüche 41 bis 44, **dadurch gekennzeichnet, daß** das Siliconharz in einer Menge von 0,1 bis 50 Gew.-%, bezogen auf das Gesamtgewicht des Stiftes, vorliegt.

46. Stift nach einem der Ansprüche 41 bis 45, **dadurch gekennzeichnet, daß** der siliconhaltige grenzflächenaktive Stoff in einer Menge von 0,1 bis 30 Gew.-%, bezogen auf das Gesamtgewicht des Stiftes, vorliegt.

47. Kosmetisches Pulver, das eine Zusammensetzung nach einem der Ansprüche 1 bis 35 enthält.

48. Pulver nach dem vorhergehenden Anspruch, **dadurch gekennzeichnet, daß** der Ester in einer Menge von 0,1 bis 23 Gew.-%, bezogen auf das Gesamtgewicht des Pulvers, vorliegt.

49. Pulver nach Anspruch 47 oder 48, **dadurch gekennzeichnet, daß** das Siliconöl in einer Menge von 0,1 bis 23 Gew.-%, bezogen auf das Gesamtgewicht des Pulvers, vorliegt.

50. Pulver nach einem der Ansprüche 47 bis 49, **dadurch gekennzeichnet, daß** die Füllstoffe in einer Menge von 0,1 bis 80 Gew.-%, bezogen auf das Gesamtgewicht des Pulvers, vorliegen.

51. Pulver nach einem der Ansprüche 47 bis 50, **dadurch gekennzeichnet, daß** die Partikelphase in einer Menge von 77 bis 99,9 Gew.-%, bezogen auf das Gesamtgewicht des Pulvers, vorliegt.

52. Pulver nach einem der Ansprüche 47 bis 51, **dadurch gekennzeichnet, daß** es als kompaktes Pulver vorliegt.

53. Verfahren zur nicht therapeutischen Behandlung der Haut und/oder der Schleimhäute und insbesondere Verfahren zum Schminken, das darin besteht, auf die Haut und/oder die Schleimhäute eine Zusammensetzung nach einem der Ansprüche 1 bis 35 und/oder ein Produkt nach einem der Ansprüche 36 bis 40 und/oder einen Stift nach einem der Ansprüche 41 bis 46 und/oder ein Pulver nach einem der Ansprüche 47 bis 52 aufzutragen.

54. Verwendung der Kombination mindestens einer Siliconverbindung und mindestens eines Esters einer Fettsäure oder eines Fettalkohols, wobei die Kohlenstoffkette des Fettalkohols oder der Fettsäure gesättigt und verzweigt vorliegt und 24 bis 28 Kohlenstoffatome aufweist, in einer kompakten kosmetischen Zusammensetzung, um die Stoßfestigkeit der Zusammensetzung zu verbessern.

55. Verwendung der Kombination mindestens einer Siliconverbindung und mindestens eines Esters einer Fettsäure oder eines Fettalkohols, wobei die Kohlenstoffkette der Fettsäure oder des Fettalkohols gesättigt und verzweigt ist und 24 bis 28 Kohlenstoffatome aufweist, zur Herstellung einer kompakten Zusammensetzung, um die Stoßfestigkeit der Zusammensetzung zu verbessern.

## Claims

1. Cosmetic composition, in particular a cosmetic make-up composition, **characterized in that** it comprises (i) at least one fatty acid ester or fatty alcohol ester, the carbon chain of the fatty acid or fatty alcohol being saturated and branched and containing 24 to 28 carbon atoms, and (ii) at least one silicone compound.

2. Composition according to Claim 1, **characterized in that** the ester comprises at least 2 branched C₂₄ to C₂₈ chains.

3. Composition according to either of the preceding claims, **characterized in that** the ester has a carbon number of greater than or equal to 50 and preferably greater than or equal to 70.

4. Composition according to any one of the preceding claims, **characterized in that** at least one of the branched chains of the ester contains 24 carbon atoms.

5. Composition according to any one of the preceding claims, **characterized in that** the ester is chosen from branched C24 fatty acid triglycerides, branched C24 fatty acid esters of pentaerythritol and branched C24 fatty alcohol esters of diacids, and mixtures thereof.

6. Composition according to any one of the preceding claims, **characterized in that** the ester has a refractive index of greater than 1.45 at 20°C.

7. Composition according to any one of the preceding claims, **characterized in that** the ester has an iodine number of less than or equal to 4.

8. Composition according to any one of the preceding claims, **characterized in that** the ester is glyceryl tri (decyl 2-tetradecanoate).

9. Composition according to any one of the preceding claims, **characterized in that** the ester is present in an amount ranging from 0.1% to 99.9% by weight relative to the total weight of the composition, and preferably in an amount ranging from 1% to 99% by weight relative to the total weight of the composition.

10. Composition according to any one of the preceding claims, **characterized in that** the silicone compound is chosen from volatile or nonvolatile silicone oils, silicone waxes, silicone gums, silicone resins and silicone surfactants, and/or mixtures thereof.

11. Composition according to any one of the preceding claims, **characterized in that** the silicone compound is present in the composition in an amount which may range from 0.1% to 99.9% and preferably from 1% to 99% by weight relative to the total weight of the composition.

12. Composition according to any one of the preceding claims, **characterized in that** the silicone compound is chosen from silicone oils whose viscosity at 25°C ranges from 0.05-15 cm²/s (5 cst to 1500 cst) and more preferably from 0.1-5 cm²/s (10 cst to 500 cst).

13. Composition according to any one of the preceding claims, **characterized in that** the silicone compound is chosen from the phenylsilicone oils corresponding to formula (II) below: 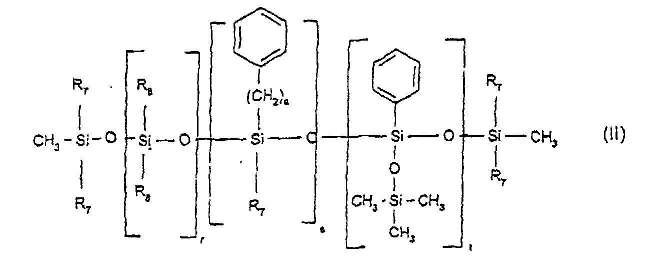 in which:
R₇ represents an alkyl radical containing from 1 to 30 carbon atoms, or an aryl radical, preferably phenyl, or an aralkyl radical,
R₈ represents an alkyl radical containing from 1 to 30 carbon atoms,
r is equal to 0 or 1,
s represents an integer between 0 and 100,
t represents an integer between 0 and 100,
a represents an integer between 0 and 10,
with the proviso that the sum r+s+t is between 1 and 100 and that if s=t=0, then R₇ represents an aryl or aralkyl radical.

14. Composition according to any one of Claims 10 to 13, **characterized in that** the volatile silicone oil is chosen from cyclic volatile silicones containing from 3 to 8 silicon atoms, cyclocopolymers of the dimethylsiloxane/methylalkylsiloxane type and linear volatile silicones containing from 2 to 9 silicon atoms.

15. Composition according to any one of Claims 10 to 14, **characterized in that** the silicone oil is present at a content ranging from 1% to 99% by weight relative to the total weight of the composition.

16. Composition according to any one of Claims 10 to 15, **characterized in that** the silicone compound is chosen from silicone waxes corresponding to formula (III) below: 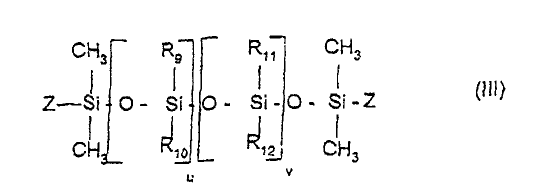 in which:
- R₉, R₁₁ and R₁₂ are, independently of each other, a C₁-C₃₀ alkyl or alkoxy radical or a C₆-C₃₀ aryl radical,
- R₁₀ is a C₂-C₃₆ alkyl or alkoxy radical or a C₂-C₃₆ alkyl or alkoxy radical substituted with an ester group, R₁₀ also possibly being a methyl radical when Z is a C₂-C₃₀ alkyl or alkoxy radical or a C₂-C₃₀ ester,
- Z is a C₁-C₃₀ alkyl or alkoxy radical, a C₆-C₃₀ aryl radical or a C₁-C₃₀ ester;
- u is 0 or an integer between 1 and 100,
- v is an integer between 1 and 100.

17. Composition according to the preceding claim, **characterized in that** the silicone wax is chosen from C₂₄-C₂₈ alkyl dimethicone, stearyldimethicone, dibehenoxydimethicone and stearoxydimethicone.

18. Composition according to the preceding claim, **characterized in that** the silicone wax is present at a content which may range from 0.1% to 50% by weight relative to the total weight of the composition.

19. Composition according to any one of Claims 10 to 18, **characterized in that** the silicone compound is chosen from silicone gums corresponding to formula (IV) below: 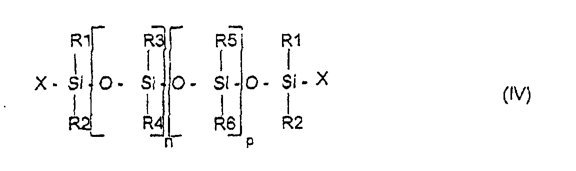 in which:
R₁, R₂, R₅ and R₆ are, independently of each other, an alkyl radical containing 1 to 6 carbon atoms,
R₃ and R₄ are, together or separately, an alkyl radical containing from 1 to 6 carbon atoms or an aryl radical and in particular phenyl,
X is an alkyl radical containing from 1 to 6 carbon atoms, a hydroxyl radical or a vinyl radical,
n and p being chosen so as to give the silicone gum a viscosity of greater than 5000 cm²/s (500 000 cst).

20. Composition according to the preceding claim, **characterized in that** the silicone gum is present at a content ranging from 0.1% to 20% by weight relative to the total weight of the composition.

21. Composition according to any one of Claims 10 to 20, **characterized in that** the silicone resin is chosen from those of formulae R₃SiO_{1/2}, R₂SiO_{2/2}, RSiO_{3/2} and SiO_{4/2}, R representing an alkyl group containing from 1 to 6 carbon atoms or a phenyl group.

22. Composition according to the preceding claim, **characterized in that** the silicone resin is present at a content which may range from 0.1% to 90% by weight relative to the total weight of the composition.

23. Composition according to any one of Claims 10 to 22, **characterized in that** the silicone surfactant is chosen from silicones comprising at least one oxyalkylenated group of the type (-CₓH₂ₓO)ₐ in which x may range from 2 to 6 and a is greater than or equal to 1.

24. Composition according to the preceding claim, **characterized in that** the silicone surfactant is present at a content ranging from 0.1% to 40% by weight relative to the total weight of the composition.

25. Composition according to any one of the preceding claims, **characterized in that** it also comprises an oil chosen from mink oil, turtle oil, soybean oil, grapeseed oil, sesame oil, corn oil, rapeseed oil, sunflower oil, cottonseed oil, avocado oil, olive oil, castor oil, jojoba oil, groundnut oil; hydrocarbon oils, such as liquid paraffins, squalane, petroleum jelly, hydrogenated polyisobutenes and polybutenes; fatty esters, such as isopropyl myristate, isopropyl palmitate, butyl stearate, hexyl laurate, isononyl isononanoate, 2-ethylhexyl palmitate, 2-hexyldecyl laurate, 2-octyldecyl palmitate, 2-octyldodecyl myristate or lactate, 2-diethylhexyl succinate, diisostearyl malate, glyceryl or diglyceryl triisostearate, perfluoro oils; higher fatty acids such as myristic acid, palmitic acid, stearic acid, behenic acid, oleic acid, linoleic acid, linolenic acid or isostearic acid; higher fatty alcohols such as cetanol, stearyl alcohol, oleyl alcohol or octyldodecanol.

26. Composition according to any one of the preceding claims, **characterized in that** it also comprises a wax chosen from beeswaxes, lanolin waxes and Chinese insect waxes; carnauba wax, candelilla wax, ouricury wax, cork fibre waxes, sugarcane waxes, Japan waxes, hydrogenated jojoba waxes and hydrogenated oils such as hydrogenated sunflower oil, hydrogenated castor oil, hydrogenated coconut oil and hydrogenated lanolin; paraffins, microcrystalline waxes, montan waxes and ozokerites; polyethylene waxes, waxes obtained by Fisher-Tropsch synthesis, copolymers and waxy esters.

27. Composition according to any one of the preceding claims, **characterized in that** it also comprises a particulate phase.

28. Composition according to the preceding claim, **characterized in that** the particulate phase comprises pulverulent compounds chosen from pigments and/or nacres and/or fillers and/or mixtures thereof.

29. Composition according to the preceding claim, **characterized in that** the pigments are chosen from titanium dioxides, of spherical or flake form, zirconium dioxides or cerium dioxides, zinc oxides, iron oxides or chromium oxides, nanotitaniums, ferric blue, carbon black, calcium salts, barium salts, aluminium salts or zirconium salts, acidic dyes such as halo-acid dyes, azo dyes or anthraquinone dyes, and pigments coated with silicone compounds such as PDMSs and/or with polymers.

30. Composition according to the preceding claim, **characterized in that** the said pigments are present in a proportion of from 0-40% by weight and preferably in a proportion of from 0.1% to 30% by weight relative to the total weight of the composition.

31. Composition according to any one of Claims 28 to 30, **characterized in that** the fillers are chosen from talc, mica, silica, kaolin, Nylon powder, poly-β-alanine powder and polyethylene powder, Teflon, lauroyllysine, starch, boron nitride, bismuth oxychloride, tetrafluoroethylene polymer powders, polymethyl methacrylate powders, polyurethane powders, polystyrene powders, polyester powders, synthetic hollow microspheres, microsponges, silicone resin microbeads, zinc oxides and titanium oxides, zirconium oxides or cerium oxides, precipitated calcium carbonate, magnesium carbonate and magnesium hydrocarbonate, hydroxyapatite, hollow silica microspheres, glass microcapsules or ceramic microcapsules, metal soaps derived from organocarboxylic acids containing from 8 to 22 carbon atoms and preferably from 12 to 18 carbon atoms, for instance zinc stearate, magnesium stearate or lithium stearate, zinc laurate or magnesium myristate, SiO₂/TiO₂/SiO₂, TiO₂/CeO₂/SiO₂ or TiO₂/ZnO/talc compounds, and polyethylene terephthalate/polymethacrylate polymers in the form of flakes.

32. Composition according to the preceding claim, **characterized in that** the fillers are present in a proportion of 0-99% by weight relative to the total weight of the composition.

33. Composition according to any one of Claims 28 to 32, **characterized in that** the nacres are chosen from natural mother-of-pearl, mica coated with titanium oxide, with iron oxide, with natural pigment or with bismuth oxychloride, and coloured titanium mica.

34. Composition according to the preceding claim, **characterized in that** the nacres are present in the composition in a proportion of from 0% to 60% by weight and more preferably in a proportion of from 0.1% to 40% by weight relative to the total weight of the composition.

35. Composition according to any one of the preceding claims, **characterized in that** it also comprises an aqueous phase in a content ranging from 0.1% to 95% by weight relative to the total weight of the composition.

36. Product for the complexion or lips comprising a composition as defined in any one of Claims 1 to 35.

37. Product according to Claim 36, **characterized in that** the wax is present in a content ranging from 0.1% to 5% by weight relative to the total weight of the product.

38. Product according to the preceding claim, **characterized in that** it is fluid.

39. Product according to any one of Claims 36 to 38, **characterized in that** the silicone resin is preferably present in a content ranging from 0.1% to 70% by weight relative to the total weight of the product.

40. Product according to any one of Claims 36 to 39, **characterized in that** the fillers are preferably present in a content ranging from 0.1% to 40% by weight and more preferably in a content ranging from 1% to 20% by weight relative to the total weight of the product.

41. Complexion stick or lipstick, **characterized in that** it comprises a product as defined in any one of Claims 36 to 40.

42. Stick according to Claim 41, **characterized in that** the ester is present in a content ranging from 1% to 85% by weight and preferably from 1% to 30% by weight relative to the total weight of the stick.

43. Stick according to Claim 41 or 42, **characterized in that** the silicone oil is present in a content ranging from 1% to 85% by weight and preferably from 1% to 30% by weight relative to the total weight of the stick.

44. Stick according to any one of Claims 41 to 43, **characterized in that** the silicone gum is present in a content ranging from 0.1% to 10% by weight relative to the total weight of the stick.

45. Stick according to any one of Claims 41 to 44, **characterized in that** the silicone resin is present in a content ranging from 0.1% to 50% by weight relative to the total weight of the stick.

46. Stick according to any one of Claims 41 to 45, **characterized in that** the silicone surfactant is present in a content ranging from 0.1% to 30% by weight relative to the total weight of the stick.

47. Cosmetic powder comprising a composition as defined in any one of Claims 1 to 35.

48. Powder according to the preceding claim, **characterized in that** the ester is present in a content ranging from 0.1% to 23% by weight relative to the total weight of the powder.

49. Powder according to Claim 47 or 48, **characterized in that** the silicone oil is present in a content ranging from 0.1% to 23% by weight relative to the total weight of the powder.

50. Powder according to any one of Claims 47 to 49, **characterized in that** the fillers are present in a content ranging from 0.1% to 80% by weight relative to the total weight of the powder.

51. Powder according to any one of Claims 47 to 50, **characterized in that** the particulate phase is present in a content ranging from 77% to 99.9% by weight relative to the total weight of the powder.

52. Powder according to any one of Claims 47 to 51, **characterized in that** it is in the form of a compact powder.

53. Non-therapeutic process for treating the skin and/or mucous membranes, in particular a make-up process, which consists in applying to the skin and/or mucous membranes a composition as defined in any one of Claims 1 to 35 and/or a product as defined in any one of Claims 36 to 40 and/or a stick as defined in any one of Claims 41 to 46 and/or a powder as defined in any one of Claims 47 to 52.

54. Use of a combination of at least one silicone compound and of at least one fatty acid ester or fatty alcohol ester, the carbon chain of the fatty acid or fatty alcohol being saturated and branched and containing 24 to 28 carbon atoms, in a compact cosmetic composition, with the aim of improving the impact strength of the said composition.

55. Use of the combination of at least one silicone compound and of at least one fatty acid ester or fatty alcohol ester, the carbon chain of the fatty acid or fatty alcohol being saturated and branched and containing 24 to 28 carbon atoms, for the preparation of a compact composition, with the aim of improving the impact strength of the said composition.
